# EUROPEAN PATENT APPLICATION

(11) **EP 2 374 463 A1**
(43) Date of publication of application: **12.10.2011**
(21) Application number: 09833586.2
(22) Date of filing: 30.11.2009
(51) Int. Cl.: A61K 33/36, A61P 35/00

(54) **COMPOSITION FOR IMPROVING RADIOTHERAPY FOR CANCER**

(30) Priority: 16.12.2008 KR 20080127828
(71) Applicant: Chonjisan Co., Ltd, Seoul 137-853 (KR)
(72) Inventor: BAE, Ill Ju, Seoul 156-761 (KR); PARK, Sae Gwang, Busan 614-807 (KR)
(74) Representative: Albrecht, Thomas
(86) International application number: PCT/KR2009/007070
(87) International publication number: WO 2010/071308

(57) **Abstract**

Disclosed is a composition for improving radiotherapy for cancer, containing tetra-arsenic oxide (As₄O₆) as an active ingredient. The disclosed composition for improving radiotherapy for cancer improves the efficiency of radiotherapy, and thus reduces side effects caused by the high-dose radiation. The disclosed composition uses tetra-arsenic oxide, the safety of which is proven, from among conventional arsenic compound derivatives, to prevent side effects caused by arsenictrioxide, taxol, cisplatin, fluorouracil, leuprolide, or the like, which are known as conventional radiosensitizers.

## Description

### Technical Field

The present invention relates to a composition for improving radiotherapy, and more particularly to a composition for improving radiotherapy for cancer, which includes an arsenic compound as an active ingredient.

### Background Art

At present, about 35% of cancer patients in Korea, and about 50% of cancer patients in US are treated with radiotherapy. In Korea, the ratio of cancer patients undergoing radiotherapy has been continuously increased, and also the importance of radiotherapy in cancer treatment has been increased.

As described above, radiotherapy is currently known to be a necessary treatment method for various kinds of cancers. However, in conventional cancer radiotherapy, a local failure is caused mainly due to radio-resistance of cancer cells, and the existence of hypoxia cells (Int. J. Radiation oncology Biol. Phys., 1988, 14, 831-833). Also, after such a failure of radiotherapy, when a high dose of radiation is irradiated by increasing a radiation dose during treatment, there is raised a problem of the occurrence of radiotherapy side-effects such as the destruction of a normal human tissue.

Accordingly, there has been continuously conducted researches on a radiotherapy enhancer and a radiosensitizing compound which can improve the efficiency of radiotherapy (Oncogene, 2004, 23, 1599-1607; Int. J. Radiation Oncology Biol. Phys. , 2006, 64(5), 1466-1474 ; Clin, Cancer Res, 2007, 13(16), 4891-4899). So far, according to reports, Taxol, cisplatin, Fluorouracil (5-Fu), Leuprolide, etc., known as antitumor agents, have been researched as radiotherapy enhancers in various solid cancers such as breast cancer, uterine cancer, gastric adenocarcinoma, ovarian cancer, lung cancer, prostate cancer, colorectal cancer, etc. Also, it was proved that when the administration of such a compound is carried out side by side with radiotherapy, the efficiency of radiotherapy is improved (Oncology Rep. 2006 16: 1085-1091; Int J Radiat Oncol Biol Phys. 2006, 1, 64 (5), 1466-1474; Clin Cancer Res. 2005, 1, 11(19), 7047-7052 ; Int J Radiat Biol. 2007, 83(1), 41-47).

However, the above mentioned antitumor agents have a problem in view of actual application because they cannot show radiotherapy improving effects in various cancer cell tissues and also cause considerable side effects.

Also, arsenic trioxide (AS₂O₃), which was used as an antitumor agent in traditional Chinese medicine, has been known to be effective in various leukemia treatments including Acute promyelocytic leukemia (APL) through long-term clinical tests, and also its radiosensitivity improving effect obtained through its use in combination with radiation was reported (Korean Patent Publication No. 2003-005819).

However, arsenic trioxide was reported to cause a serious gastrointestinal toxicity when it is administered through an oral dosage form (with the highest simplicity and low side-effects) (Z.X. Shen et al., Use of arsenic trioxide (AS2O3) in the treatment of acute promyelocytic leukemia (APL): II. Clinical efficacy and pharmacokinetics in relapsed patients. Blood 89 (1997) 3354-60). Accordingly, it is required to develop a novel effective radiotherapy enhancer with low side-effects.

Meanwhile, another arsenic compound, tetra-arsenic oxide (Tetras, AS₄O₆; 2,4,6,8,9,10-Hexaoxa-1,3,5,7-tetraarsatricyclo [3.3.1.1^{3,7}]decane), is a compound which is significantly different from arsenic trioxide in view of chemicophysical properties. Arsenic trioxide has an unlimited second dimensional sheet structure while tetra-arsenic oxide has an adamantyl structure (K.A. Becker et al., The polymorphic modification of arsenic trioxide. Prog. Inorg. Chem. 4 (1962) 1-72).

Such tetra-arsenic oxide is known to show a strong antitumor effect on various cancers such as uterine cancer, lung cancer, maxillary sinus cancer, renal cell carcinoma, bladder cancer, with a very low side effect (Korean Patent Publication No. 1999-0084594). However, whether or not the tetra-arsenic oxide has an effect, as a radiotherapy enhancer, has not yet been reported.

### Disclosure

### Technical Problem

Therefore, the inventors of the present invention have conducted research to develop a radiotherapy enhancer which has an effect for improving radiotherapy on cancer and can be used in combination with radiotherapy without a side effect. They have completed the present invention by finding at the very first that tetra-arsenic oxide (a kind of arsenic compound) has an effect for improving radiotherapy on cancer.

Accordingly, an object of the present invention is to provide a composition including tetra-arsenicoxide showing an improving effect of radiotherapy on cancer.

### Technical solution

In order to achieve the object, the present invention provides a composition for improving radiotherapy on cancer, which includes tetra-arsenic oxide as an active ingredient.

Hereinafter, the present invention will be described in more detail.

The inventors of the present invention have conducted research to develop a low-toxicity and low-side effect radiotherapy enhancer, which has little gastrointestinal toxicity in oral administration unlike the conventionally used arsenic trioxide, and then found that at the very first, tetra-arsenic oxide (an arsenic compound conventionally known to have a very low toxicity) surprisingly shows an improving effect of radiotherapy on cancer.

Accordingly, the present invention provides a composition for improving radiotherapy on cancer, which includes tetra-arsenic oxide as an active ingredient.

Tetra-arsenic oxide included in the composition for improving radiotherapy may be directly prepared by a person skilled in the art according to a method widely known in the art (Korean Patent Publication No. 1999-0084594).

For radiotherapy on cancer, there is no specific limitation in the cancer to which the radiotherapy improving composition can be applied. In other words, the composition may be applied to all cancers applicable to radiotherapy, such as uterine cancer, lung cancer, breast cancer, nasopharyngeal squamous cell carcinoma, encephaloma, esophageal cancer, liver cancer, renal cell carcinoma, maxillary sinus cancer, bladder cancer, prostate cancer, melanoma, etc.

The fact that the inventive composition shows an improving effect of radiotherapy on cancer is proved through the Examples below. Specifically, in a case of a mouse model having rodent fibrosarcoma, the administration of tetra-arsenic oxide in combination with irradiation significantly reducedan increase in the fibrosarcoma size, compared to irradiation alone.

Also, in a case of a mouse model having human pharynx squamous cell carcinoma, when tetra-arsenic oxide is administered alone in a small amount causing no side effect on a normal cell, a cancel cell size is hardly reduced compared to that in a control group. Meanwhile, when such administration of tetra-arsenic oxide in a small amount is carried out in combination with radiotherapy, a significantly high tumor size reducing effect was achieved, compared to that in treatment of radiation alone.

Accordingly, it can be said that when tetra-arsenic oxide is administered in an amount causing no damage to a normal tissue during radiation treatment, it is possible to significantly lower the radiation dose required for achieving the same cancer therapeutic effect, and to show an ideal anticancer effect for significantly reducing the side effects of normal cells. In other words, the administration of tetra-arsenic oxide as a radiosensitizer can reduce a radiation's side-effect on a normal cell which is one problem of radiotherapy. Furthermore, when a cancercell shows a radio-resistance, it is possible to increase a radiation's anticancer effect with no increase in the radiation dose.

The radiotherapy improving composition according to the present invention may be administered during radiotherapy. Otherwise, the administrationof the radiotherapy improving compositionmay be carried out before or after radiotherapy with a time interval as long as the radiotherapy and the inventive radiotherapy improving composition shows interactive effects.

The inventive radiotherapy improving composition may be formulated in conventional pharmaceutical formulations known in the art. The pharmaceutical formulations include oral preparations, injections, suppositories, transdermal drugs, and pernasal drugs. However, the present invention is not limited thereto, and the composition may be formulated and administered in any other formulation.

The oral administration of tetra-arsenic oxide shows a very low toxicity (M.J. Park et al., Tetra-arsenic oxide, a novel orally administrable angiogenesis inhibitor. Int. J. Oncol. 22, 2003, 1271-6), and thus the inventive radiotherapy improving composition can be orally administered without a concern for causing side effects. Accordingly, the inventive radiotherapy improving composition may be preferably formulated in oral dosage formulations such as liquid formulation, suspension, powders, granules, tablets, capsules, pills, or extracts.

When the composition is formulated in each of the above mentioned formulations, pharmaceutically acceptable carriers or additives required for the preparation of each formulation may be added. Representatively, for the formulation of an oral dosage formulation, as the carrier, at least one of a diluent, a lubricant, a binding agent, a disintegrating agent, a sweeting agent, a stabilizer, and a preservative may be selected and used. Also, as the additive, at least one of flavors, vitamins, antioxidants may be selected and used.

There is no limitation in the carrier and additive as long as they are pharmaceutically acceptable. Specifically preferably, examples of the diluent may include lactose, corn starch, soybean oil, microcrystalline cellulose, and mannitol, examples of the lubricant may include magnesium stearate and talc, and examples of the binding agent may include polyvinyl pyrrolidone and hydroxypropylcellulose. Also, examples of the disintegrating agent may include carboxymethylcellulose calcium, sodium starch glycolate, polacrilin potassium, and crospovidone, examples of the sweeting agent may include white sugar, fructose, sorbitol, and aspartame, examples of the stabilizer may include sodium carboxymethyl cellulose, beta-cyclodextrin, bee wax, and xanthan gum, and examples of the preservative may include methyl ρ-Hydroxybenzoate, propyl p-Hydroxybenzoate, and potassium sorbate.

Also, besides the above mentioned components, in order to improve taste, conventionally known additives such as natural aromatics (e.g., Japanese apricot aromatic, lemon aromatic, pineapple aromatic, herb aromatic), natural coloring agents (e.g., natural fruit juice, chlorophyllin, flavonoid), sweetness components (e.g., fructose, honey, sugar alcohol, sugar) and acidifiers (e.g., citric acid, sodium citrate) may be used in combination.

Technologies required for such formulation and pharmaceutically acceptable carriers and additives are widely known to a person in the formulation art, and may refer to Remington's Pharmaceutical Sciences (19th ed.,1995).

The radiotherapy improving composition including tetra-arsenic oxide as an active ingredient is administered in concurrence (combination) with irradiation in order to achieve a radiotherapy improving effect on cancer. Herein, the tetra-arsenic oxide may be administered in an amount of 0.1 to 10 mg/kg/day once or several times a day, and the total dose of radiation may range 1 to 100Gy at once or in stages.

The administration period of tetra-arsenic oxide may be appropriately increased/decreased according to the kind of cancer, the process of cancer, the administration method, the sex, age, and weight of the patient, etc. Herein, the tetra-arsenic oxide may be administered before/after radio the rapy, andalsomaybedailyregularly administered in a fixed amount, or concentratively administered in a large amount for a short time.

In the radiotherapy, from among conventionally different techniques, external beam radiation generated from radiation of a cobalt-60 raw material, linear accelerator, and a proton, neutron, or hadron beam raw material is administered. Irradiation is carried out in a period of 0 to 7 days, and after ward, tetra-arsenic oxide is administered. Preferably, irradiation is carried out in a period of 0.5 to 24 days, and afterward, tetra-arsenic oxide is administered. Simultaneous radiotherapy includes cumulative external irradiation on a patient in a radiation dose of 1 to 100Gy. The radiation dose preferably ranges from 1 to 60Gy. The external radiation dose is administered in 1 to 60 fractional doses, preferably in 5 to 30 fractional doses. Preferably, the fractional doses may be administered for 1 to 26 weeks.

In the present invention, it is understood that in the term of "fractional doses", the total activity of fractional doses (necessarily) increases a cumulative external irradiation obtained by the administration of one radiation dose.

### Advantageous Effects

As described above, the inventive composition for improving radiotherapy on cancer, which includes tetra-arsenic oxide as an active ingredient, may be used in combination with radiotherapy, in order to improve a radiotherapy effect. Accordingly, it is possible to improve the efficiency of radiotherapy on cancer showing radio-resistance.

Also, through such a radiotherapy improving effect, it is possible to reduce a radiation dose for radiotherapy. Further, in view of therapeutic efficiency, it is possible to achieve a high anticancer effect which can be obtained by irradiation of a high radiation dose. Thus, there is an advantage in that it is possible to reduce side-effects caused by irradiation of a high radiation dose in radiotherapy.

Furthermore, from among conventional arsenic compound derivatives, tetra-arsenic oxide which has proved to have stability is used as a raw material in the present invention. Thus, it is possible to solve various kinds of side-effects generated by conventionally used radio the rapy enhancers such as arsenic trioxide, Taxol, cisplatin, fluorouracil, or Leuprolide.

### Brief Description of the Drawings

The foregoing and other objects, features and advantages of the present invention will become more apparent from the following detailed description when taken in conjunction with the accompanying drawings in which:
FIG. 1 is a graph showing a change in tumor volume when a local tumor region of Balb/c mice having Meth-A tumor on their rear legs is irradiated in combination with abdominal cavity administration of tetra-arsenic oxide, or is independently irradiated;
FIG. 2 is a graph showing a change in tumor volume when Balb/c nu/nu mice having nasopharyngeal squamous cell carcinoma are divided into 4 groups, and their local tumor region is irradiated in combination with abdominal cavity administration of tetra-arsenic oxide, or is independently irradiated; and
FIG. 3 is a graph showing a change in tumor volume when the example is carried out in the same manner as described in FIG. 2 except that fractional irradiation was carried out.

### <description on main marks of drawings>

○ : control group
● : independent administration of tetra-arsenic oxide
■, □ : independent irradiation
▼, ∇ : combination of irradiation with tetra-arsenic oxide administration

### Best Mode

### Mode for Invention

Hereinafter, a pharmacological effect of tetra-arsenic oxide of the present invention will be described in more detail with reference to Examples below.

### <Example>

### A. experimental animal and tumor model preparation

A mouse and a tumor cell line were bought as described below, and then a tumor model was prepared.

Balb/c, and Balb/c, nu/nu female mice (weight: 20 to 25g, age: 6 to 8 weeks) were obtained from Orient bio (Taejun, Korea), and WEHI164 tumor cell line (derived from Meth-A-induced fibrosarcoma) and FaDu tumor cell line (derived from human nasopharyngeal squamous cell carcinoma) were bought from KCCB (Seoul, Korea). WEHI164 cells (5 x 10⁵) or FaDu cells (1 x10⁶) in MEM 50µℓ were hypodermically injected into a rear leg of a Balb/c or Balb/c nu/nu mouse, and then the growth of a tumor was observed. All processes and treatments on animals were carried out under the guideline proved by Animal Care and Use Committees of Inje university medical college.

### B. Preparation of tetra-arsenic oxide (As₄O₆) sample liquid

Tetra-arsenic oxide obtained from Genew Science (Seoul, Korea) was placed in distilled water, and continuously stilled and dissolved. The resultant liquid was stored as a stock liquid at 4°C. The stock liquid was diluted with a phosphoric acid buffer solution in such a manner as to have a final concentration of 0.5 mg/ml.

In order to minimize acute arsenic toxicity, dextrose was added with a final concentration of 5% (F.X. Reichl,H. Kreppel, L. Szinicz, B. Fichtl, and W. Forth, Reduction of arsenic trioxide toxicity in mice by repeated treatment with glucose. Arch Toxicol Suppl 14 (1991) 225-7).

### C. irradiation and tetra-arsenic oxide treatment on a Balb/c mouse having a Meth-A tumor

In order to determine if tetra-arsenic oxide can be acted as a radiosensitizer in combination with local irradiation, for tumor reduction, the following process was carried out.

In irradiation, a cobalt-60 unit (Theratron 780; AECL, Kanata, Ontarino, Canada) was used. A local tumor region of a rear leg of a Balb/c mouse having Meth-A tumor was irradiated once, and then after 1 hour, 8mg/kg of tetra-arsenic oxide was administered once into an abdominal cavity (triangle) or not administered (square).

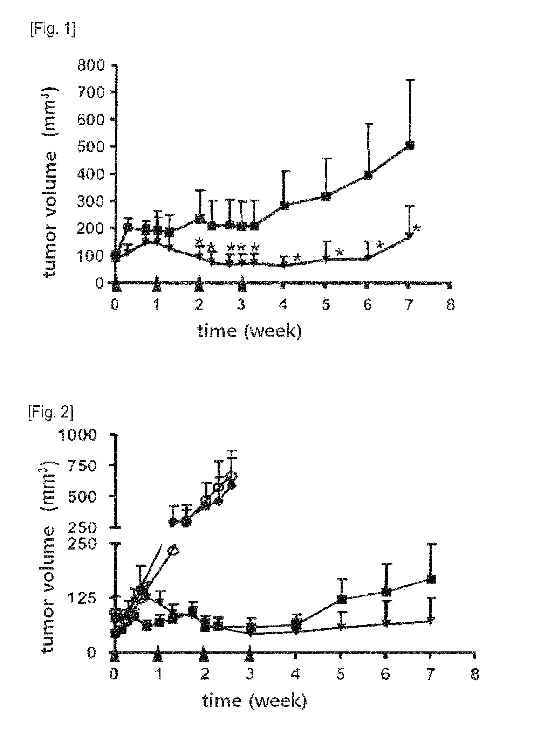

A change in the tumor size was measured by calipers twice to three times per week. A tumor volume was calculated by a x b x h/2 (a= minimum length, b= maximum length, h= height) . From respective values obtained from 6 mice, an average s.d. was obtained (* p< 0.05). The result is shown in FIG. 1.

According to the result in FIG. 1, compared to only irradiation once (square), administration of tetra-arsenic oxide in combination with irradiation (triangle) significantly reduced the growth of tumor.

### D. irradiation and tetra-arsenic oxide treatment on a Balb/c mouse having human nasopharyngeal squamous cell carcinoma

In order to determine if tetra-arsenic oxide can be synergically acted in combination with local irradiation, for tumor reduction, the following process was carried out.

Balb/c nu/nu mice having human nasopharyngeal squamous cell carcinoma on their rear legs were divided into 4 groups. To a control group, dextrose 5% aqueous solution was administered (opened circle) . Meanwhile, for the other 3 drug administration groups, irradiation was independently carried out once (square), tetra-arsenic oxide (8mg/kg) was independently administered once through an abdominal cavity (closed circle), and irradiation was carried out in combination with the administration of tetra-arsenic oxide (triangle), respectively.

A tumor volume was measured twice to three times per week. The tumor size was measured by calipers, and the tumor volume was calculated by a x b x h/2 (a= minimum length, b= maximum length, h= height) . From respective values obtained from 6 mice, an average s. d. was obtained. The result is shown in FIG. 2.

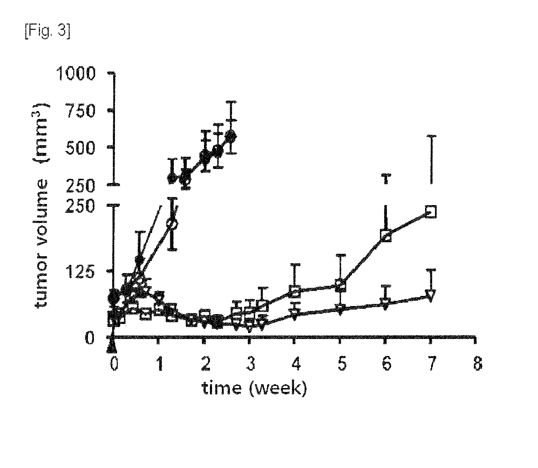

Additionally, another example was carried out in the same manner as described in FIG. 2 except that fractional irradiation was carried out in a dose of 12Gy 4 times per week, for 4 weeks. From respective values obtained from 6 mice, an average s.d. was obtained. The result is shown in FIG. 3.

According to the results shown in FIGs. 2 and 3, the separate treatment of tetra-arsenic oxide shows a similar result to a control group, and thus it is found that tetra-arsenic oxide at the concentration hardly has an effect on tumor growth inhibition. However, tetra-arsenic oxide in combination with irradiation showed a normal therapeutic effect by significantly reducing tumor growth, compared to irradiation alone. Accordingly, it can be said that tetra-arsenic oxide in combination with irradiation shows a synergic action on inhibition of tumor growth, and thus is significantly effective as a radiosensitizer. Furthermore, as shown in FIG. 3, even in a case of irradiation alone, tetra-arsenic oxide showed a significant effect as a radiosensitizer. Thus, it can be said that it is possible to improve an anticancer effect of radiation independently of irradiation protocol.

Also, since such a synergic action is achieved at a concentration where tetra-arsenic oxide can hardly inhibit tumor growth, it can be said that tetra-arsenic oxide can increase the efficiency of radiotherapy at a very low concentration with no toxicity.

Accordingly, tetra-arsenic oxide can be used in cancer treatment with minimized side-effects in radiotherapy on cancer having radio-resistance and low radio-sensitivity.

### Industrial Applicability

Although several exemplary embodiments of the present invention have been described for illustrative purposes, those skilled in the art will appreciate that various modifications, additions and substitutions are possible, without departing from the scope and spirit of the invention as disclosed in the accompanying claims.

## Claims

1. A composition for improving radiotherapy on cancer, which comprises tetra-arsenic oxide As₄O₆ as an active ingredient.

2. The composition as claimed in claim 1, which is administered before or after irradiation.

3. The composition as claimed in claim 1 or 2, which is formulated in oral dosage formulations.

4. The composition as claimed in claim 3, wherein the oral dosage formulations comprise liquid formulation, suspension, powders, granules, tablets, capsules, pills, or extracts.
